# EUROPEAN PATENT SPECIFICATION

(11) **EP 2 739 247 B1**
(45) Date of publication and mention of the grant of the patent: **10.10.2018**
(21) Application number: 12821780.9
(22) Date of filing: 03.08.2012
(51) Int. Cl.: A61F 2/24

(54) **PERCUTANEOUS HEART VALVE DELIVERY SYSTEMS**
PERKUTANE HERZKLAPPENFREISETZUNGSSYSTEME
SYSTÈMES DE POSE DE VALVULE CARDIAQUE PERCUTANÉE

(30) Priority: 05.08.2011 US 201161515679 P; 29.06.2012 US 201261666657 P
(43) Date of publication of application: 11.06.2014
(73) Proprietor: California Institute of Technology, Pasadena, CA 91125 (US)
(72) Inventor: KHERADVAR, Arash, Lirvine, CA 92617 (US); KELLEY, Gregory, S., Santee, CA 92071 (US); GHARIB, Morteza, Pasadena, CA 91125 (US)
(74) Representative: Murgitroyd & Company
(86) International application number: PCT/US2012/049645
(87) International publication number: WO 2013/022798

(56) References cited:
- WO-A2-2005/037361
- US-A1- 2005 101 988
- US-A1- 2007 293 935
- US-A1- 2007 299 499
- US-A1- 2008 039 863
- US-A1- 2009 030 497
- US-A1- 2009 099 640
- US-A1- 2009 192 585
- US-A1- 2009 192 585
- US-A1- 2011 040 374
- US-A1- 2011 040 374

## Description

### FIELD

The invention relates to medical devices for implant delivery. More particularly, the invention relates to systems allowing for stented valve delivery, repositioning and/or percutaneous retrieval.

### BACKGROUND

Correct valve positioning is crucial for treatment success and optimal outcomes after transcatheter valve implantation. For example, to maintain a stable and correct lengthwise position with the aortic annulus, a stepwise deployment that allows the valve to be repositioned both circumferentially and in axial direction (i.e., towards the left ventricle (LV) or the ascending aorta) is essential.

However, most of the current technologies are limited by instant deployment, and once the valve is deployed, repositioning and/or percutaneous retrieval is not possible- or at least difficult or potentially problematic. Too high/proximal placement of the stented valve can totally or partially obstruct the coronary ostia in a case of aortic implantation, which may result in myocardial infarction or ischemia, respectively. Additionally, if the valve is placed too high in the aorta, it may embolize into the aorta causing significant paravalvular regurgitation. On the other hand, too low/distal implantation is accompanied by compression of atrioventricular (AV) node in the membranous septum, which leads to conduction abnormalities.

Further technical developments with a focus on a repositionable and/or percutaneously retrievable valve design allow optimal placement and may thereby significantly reduce the risk of paravalvular aortic regurgitation, myocardial infarction or ischemia related to mis- or mal-positioning.
US 2009/0192585 discloses a delivery device comprising an elongate sleeve, a stent frame positioned over the sleeve, a plurality of arms passing through a port in the sleeve and releasably engaged with the stent frame. The arms expand the stent when the arms are advanced relative to the sleeve.

US 2011/0040374 discloses a device for percutaneous implantation of a replacement heart valve, wherein said device allows reposition and removal of said replacement heart valve.

### SUMMARY

The embodiments described herein address the need for improved catheter devices for coordinated delivery, repositioning and/or percutaneous retrieval of the percutaneously implanted heart valves. The delivery system apparatus is a tool that may incorporate a guide wire lumen. As such, a given device may be suitable for so-called "over-the-wire" use and include a delivery sheath covering and restraining the stent frame of the valve. Alternatively, the delivery device may be tracked trough a catheter serving such function, as in a so-called "guide" or "delivery" catheter.

In one example, the delivery apparatus includes a number of arms (such as, but not limited to three) embedded within its body that hold the valve's stent during the delivery procedure when it is collapsed. The arms are equipped with adjustable springs that are controllable by the operator from remote, and allow for robust radial expansion or deployment of the collapsed stent in increments.

In use, the arms remain attached to the valve stent frame until the stent frame is fully deployed. If the stent/stent frame is not properly deployed, the arms, which are still releasably attached to the stent until intended release, can be used for partial contraction of the stent for repositioning purposes. When the stented valve is properly positioned as desired within the heart, the arms will be released from the stent, return to their embedded/retracted positions within the apparatus. Then the entire apparatus is retracted. It may be retracted from the heart or vasculature over any guide wire used and/or through any delivery catheter employed for site access.

In one system embodiment allowing for stented valve delivery, repositioning and/or percutaneous retrieval, draw line filaments are positioned through the distal end of a pusher sleeve/draw tube, along a sleeve/tube lumen, out through sleeve/tube holes, out through proximal frame holes, along the surface of a heart valve frame, optionally in through distal frame holes, in through the distal end of the sleeve/tube, along the sleeve/tube lumen, and out the proximal end of the sleeve/tube. Variations on this approach are possible.

The draw lines may comprise polyester (PE), PTFE, suture material or another high strength (and preferably biocompatible fiber) braid or bundle of fibers such as ultrahigh-molecular-weight polyethylene (UHMWPE, sometimes shortened to UHMW). In this embodiment, and others described herein, the heart valve frame may comprise superelastic NiTi alloy heatset in a desired shape, it may be constructed of a so-called "engineering plastic" such as polyetheretherketone (PEEK) or may be constructed otherwise. Various surface treatments or finishes may be desirable. In the case of a NiTi (Nitinol) or another metallic material implant, an electro-polished surface may be preferred.

Collapsed and expanded states of a heart valve can be controlled by varying the position and/or tension applied to the draw lines. A customized handle may be provided for user interface. Draw line tension can be increased until the heart valve frame is fully collapsed and fully releasing the draw line tension allows the self-expanding heart valve frame to fully expand. The heart valve frame may be put in an intermediate state by varying the tension applied to the draw lines.

In yet another delivery system example, allowing for delivery, repositioning and/or percutaneous retrieval, different means are provided to control the state of device deployment (variably, from fully collapsed to fully expanded) of the proximal end of a self-expanding heart valve device. Such means pertains to the use of multiple sleeve or sheath features (herein optimally referred to as "zip tube" parts or an assembly with "zip tube" sheaths or fingers) provided to mechanically change an angle between adjacent strut elements and thereby the proximity of the struts. In use, the zip tube sheaths/fingers collapse the heart valve frame by "zipping" the struts into closer proximity.

In this example, the ends of a self-expanding heart valve frame are configured with a link feature. A self-expanding retainer is constructed and configured with diametrically collapsible retainer arms or fingers. A zip tube part or assembly with diametrically expandable/collapsible sheath fingers is configured in such a manner to allow the zip tube fingers to slide over the retainer fingers. The ends of the retainer fingers are configured with a clasp/link feature so as to mate to the heart valve frame clasp/link features.

The zip tube assembly may be partially advanced (distally) to trap the heart valve frame and retainer such that they will not unlink because the inner diameter (or inner dimension(s)) of the zip tube fingers are constructed so as to constrain the linked heart valve frame and retainer from unlinking when positioned around the linked frame/retainer. With the retainer serving as a means to secure the valve in position, the zip tube assembly may be variably advanced (relative to the linked heart valve frame/retainer) to variably (e.g., partially) collapse the proximal end of the heart valve device or fully advanced to fully collapse the proximal end of the heart valve device.

The zip tube part assembly may be variably retracted to allow the proximal end of the self-expanding heart valve device to variably (partially) expand or retracted sufficient to allow the self-expanding heart valve device to fully expand. Alternatively, the zip part/assembly may be secured in position and the retainer may be variably retracted to variably collapse the proximal end of the heart valve device up to fully collapsed or variably advanced to allow the self-expanding heart valve device to variably expand up to fully expanded. The zip tube part/assembly can be fully retracted allowing the heart valve frame and retainer to unlink thereby releasing the heart valve device from the delivery system so that the heart valve device may be left in position and the delivery system may be removed.

The subject delivery devices are all included within the scope of the present disclosure. Some aspects of the same are described above; more detailed discussion is presented in connection with the figures below.

The invention provides a medical device for stent delivery as defined in the appended claims.

### BRIEF DESCRIPTION OF THE DRAWINGS

The figures diagrammatically illustrate inventive embodiments. Variations other than those shown in the figures are contemplated as described in a broader sense per the Summary above, as generically claimed, or otherwise.
Figs. 1A-1F are perspective views of a stent frame and valve in various stages of deployment as may be employed in connection with the examples described herein.
Fig. 2A is a detail view of the delivery device sleeve of one example showing the location of one of a plurality of embedded arms.
Fig. 2B is a detail view illustrating the arm at the location in Fig. 2A connected to a spring system for controlling stent frame deployment.
Fig. 3 is a system overview showing the arms releasably attached to a stent frame.
Fig. 4A is a detail view depicting the arms fully extended from the delivery apparatus and Fig. 4B is a detail view depicting a hollow deployment arm with strings inside and a pull/push mechanism inside the guide tube/sleeve.
Figs. 5A-5E illustrate progressive stages of stent frame deployment and recapture for an embodiment.
Figs. 6A-6C show side, end and perspective views, respectively, of the delivery device sleeve of the embodiment shown in Figs. 5A-5E.
Figs. 7A and 7B are side views of the sent frame associated with the delivery device sleeve in contracted and expanded states, respectively.
Figs. 8A shows a variation of the subject stent frame and Fig. 8B shows a variation of the subject delivery sleeve with associated draw line filaments.
Figs. 9A-9C are side, perspective and end views, respectively, of the components in Figs. 8A and 8B assembled together.
Figs. 10A and 10B are side and end views, respectively of the same assembled components shown in a compressed state.
Figs. 11A and 11B are partial perspective and detail side views, respectively, of a stent frame for for another example.
Fig. 12 is a perspective view of a frame retainer with retainer fingers.
Figs 13A and 13B are perspective and end views, respectively, of a zip tube part or assembly and zip tube fingers.
Fig. 14A illustrates segments of an expanded heart valve frame, retainer fingers, and zip tube fingers as associated in the example and Fig. 14B shows a complete assembly of the example including these subcomponents.
Figs. 15A-15F are detail side views illustrating operation of elements within the example.

### DETAILED DESCRIPTION

Various exemplary embodiments are described below. Reference is made to these examples in a non-limiting sense, as it should be noted that they are provided to illustrate more broadly applicable aspects of the devices, systems and methods. Various changes may be made to these embodiments without departing from the scope of the various embodiments. In addition, many modifications may be made to adapt a particular situation, material, composition of matter, process, process act(s) or step(s) to the objective(s), scope of the present invention. All such modifications are intended to be within the scope of the claims made herein.

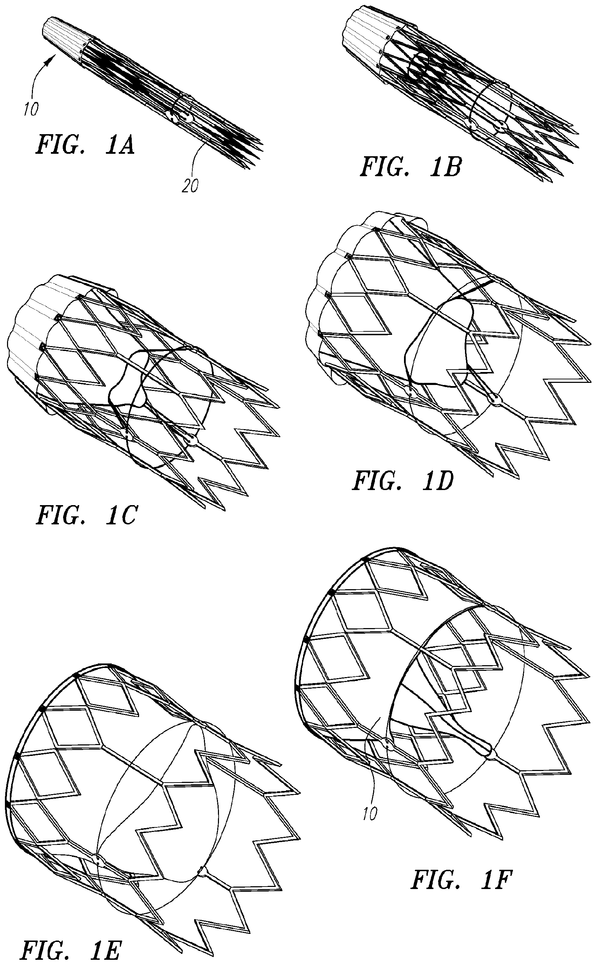

Figs. 1A-1F illustrate a suitable approach to a valve 10 attachment and its manipulation for delivery in coordinated use with an expandable stent frame 20. Further details as to valve construction and/or its manipulation for delivery may be appreciated in review of USPN 8,133,270 to Kheradvar, et al., incorporated by reference herein in its entirety. Features of the stent frame elaborated upon below in the various embodiments may be added to those shown in Figs. 1A-1F or used in connection with other suitable stent frame and/or other valve architectures.

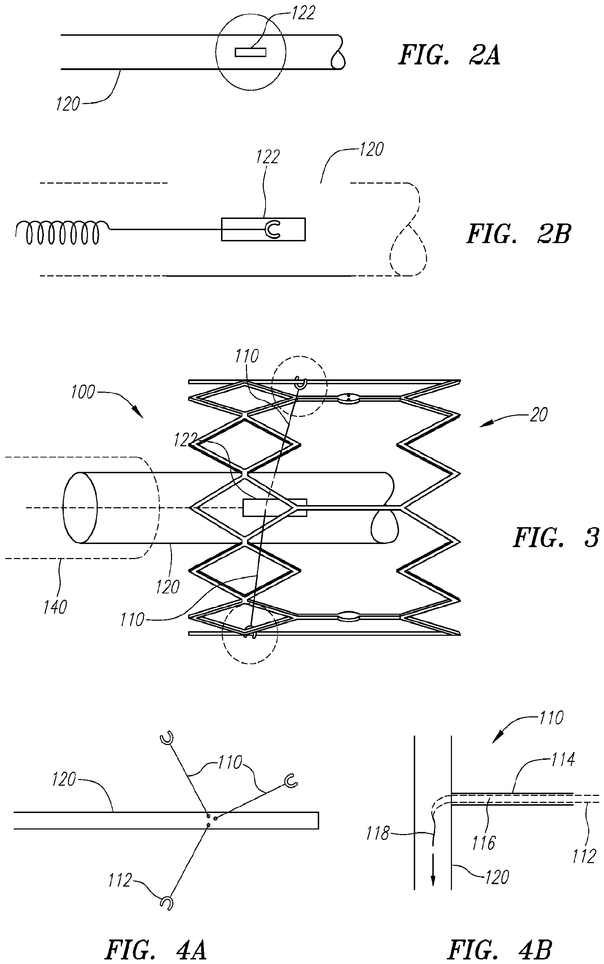

In any case, the implant (i.e., valve 10 and stent frame 20) is directly applicable for coordinated use with a delivery system as shown in Figs. 2A-4B. More specifically, a delivery system apparatus for controlled deployment of a stented heart valve system in increments is shown. The system provides for repositioning a stented heart valve system during and after deployment. As variously illustrated, device 100 includes a plurality of deployable arms 110. These are adjustably deployable. The arms are first embedded inside the apparatus. Fig. 2B illustrates the location of one of the embedded arms 110 within a delivery device sleeve 120. For tracking to the target site, the arms are hidden. The arms exit the sleeve through ports or slots 122 in the wall of the sleeve. The arms lengths are adjustable and releasably attached to the stent of the stented valve. As shown in Fig. 2B, each arm may be equipped with an in-line adjustable spring that is controllable by the operator from remote. As illustrated in Fig. 3 such actuation allow for robust radial expansion or deployment of the collapsed stent frame in increments.

The arms remain attached to the stent until the stent is fully deployed. During tracking to a site for deployment, the stented valve may be covered by a sheath incorporated in the delivery system or pass within a delivery catheter (either case illustrated by an optional sleeve 140). If the stent is not properly deployed, the arms, which are still releasably attached to the stent, can be used for partial contraction of the stent for repositioning purposes. When the stented valve is properly positioned within the heart, the arms will be released from the stent, and return to their embedded positions within the apparatus. Then the apparatus will be retracted into the sheath or through the delivery catheter from the heart or vasculature.

As seen in Fig. 4A in which the stent frame is detached, each arm may terminate in a releasable hook/jaw/clevis 112 for such purpose(s). The connection and release may be provided by a simple snap fit. Otherwise it may be provided by a more active means for stent frame interface as illustrated in Fig. 4B showing an arm comprising a hollow micro tube/sheath 114 with spring loaded strings/filaments 116 inside where a string/filament 118 inside the guide tube/sleeve 120 can be used to control the closing and opening of the hooks 112.

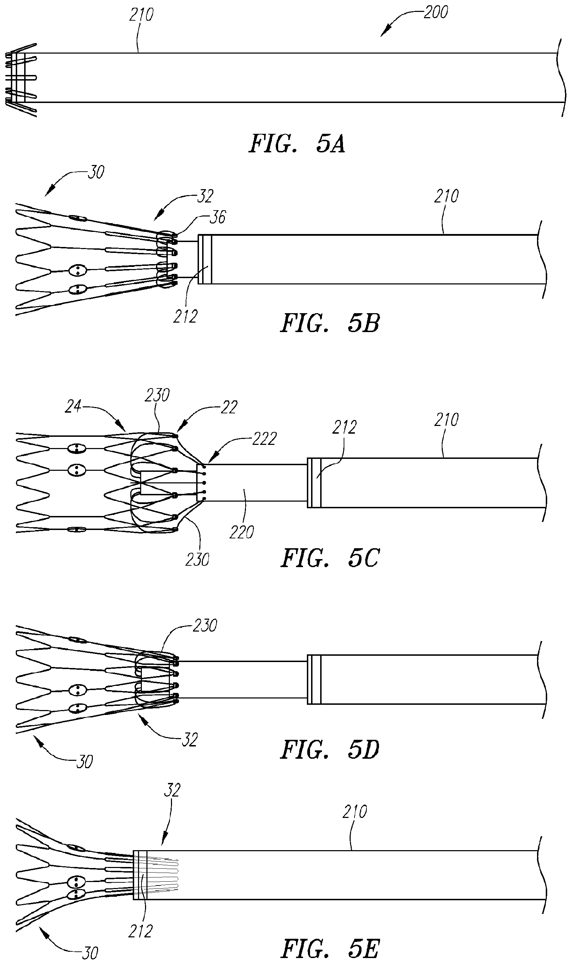

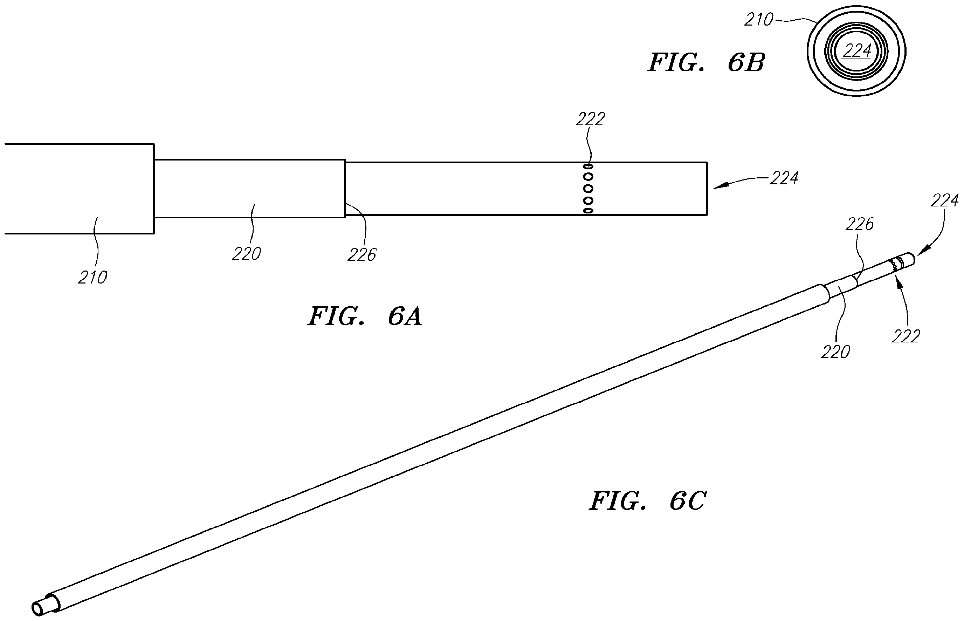

Figs. 5A-5E illustrate progressive stages of implant deployment and recapture for an embodiment. Here, in a system pictured for over-the-wire tracking to its deployment site, a delivery system 200 includes a sheath 210 (with distal radiopaque marker 212) coaxial with a pusher sleeve 220. A distal portion of sleeve 220 includes apertures 222 through which filaments 230 pass into and proximally within the length of the sleeve. The filaments loop from these apertures through proximal sent frame apertures 22 and distal stent frame apertures 24 (or alternatively past strut junctions in a different stent configuration) and into a distal end 224 of the sleeve (or a second set of distal apertures -- not shown -- in the sleeve if so-desired). Such details of the sleeve are shown un-obscured in Figs 6A-6C as well as an optional shoulder 226 for abutting proximal end/crown sections 26 of the stent frame and guide sheath 210 of the proximal end/crowns of the stent frame.

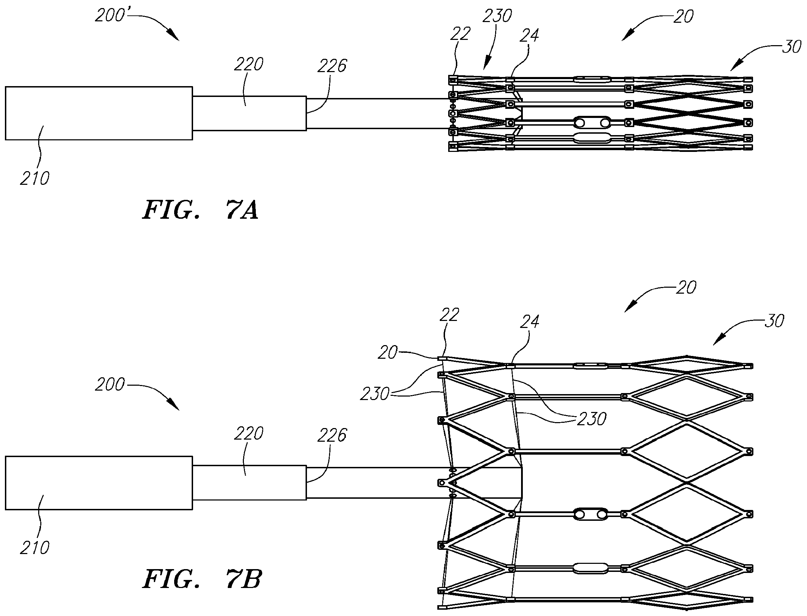

Regarding interaction between the stent frame and delivery system 200, Figs. 7A and 7B provide side views of the stent frame associated with the delivery device sleeve in contracted and expanded states, respectively. Here, the manner of stent frame expansion and contraction as related to extended filament 230 length is clearly visible.

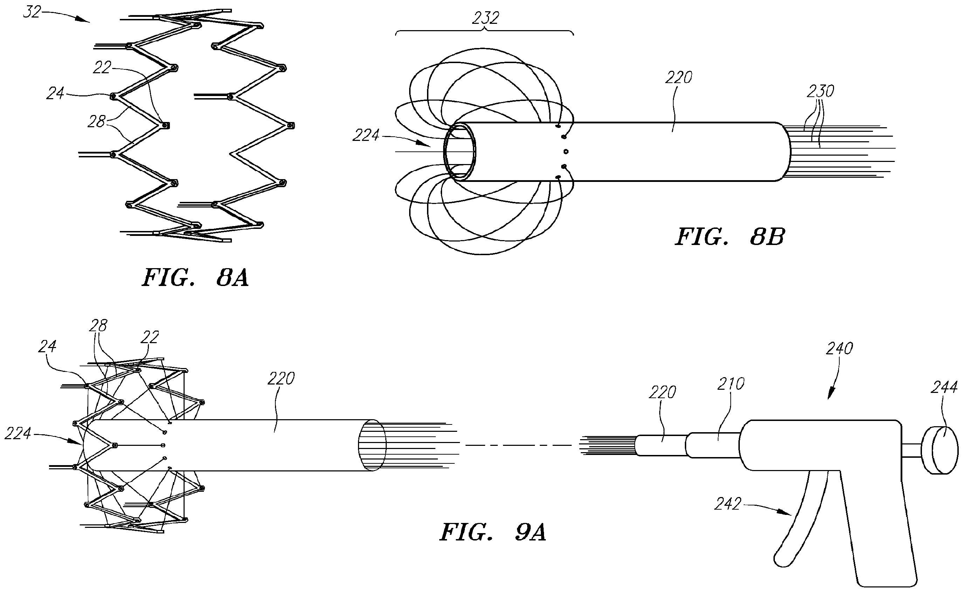

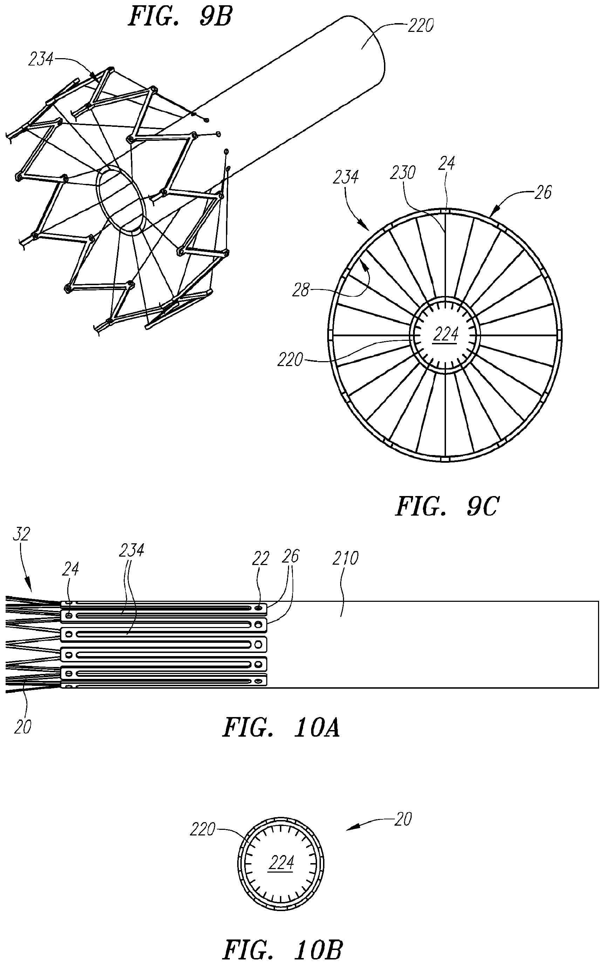

Figs. 8A and 8B further illustrate such details as described above. When assembled in a delivery system 200, stent frame 20 will be captured within loops 232. The assembled relation of elements is shown in each of Figs. 9A-9C and Figs.10A and 10B. Comparing Figs. 9A-9C to Figs. 10A and 10B, the state of the stent frame is changed from open/expanded in the former trio of figures, to compressed in the latter pair.

Such control is achieved by remote actuation of the loop filaments with a customized handle or other user interface means. Any handle may include means for group control of the filaments and independent control of sheath position. Such a handle 240 may include separate "grip" 242 and "plunger" or "slide" 244 interfaces as exemplarily illustrated in Fig. 9A for such purposes. Otherwise, mechanism internal to the handle can automate all of the various control procedure(s) by actuating a grip 242, alone.

Figs. 9A and 9B also offer good illustration of the manner in which filaments 230 pass through apertures 22, 24 and run along interposed strut sections 28. Fig. 9C illustrates the radial relationship of the apertures and filament 230 portions. Here, a crossing segment 234 of the filament between the apertures 22 and 24 is positioned outside of and opposing strut section 28. The crossing segments are angled with the struts when the stent frame is in an expanded state an more close to axially aligned when the stent is compressed as shown in Figs. 10A and 10B.

As noted above, the transition between the open and compresses (and states therebetween) is managed by letting-out or reeling-in the draw line filament determining the size of the control loop. Ultimately, one end of the line is pulled all of the way through the stent aperture to finally release the implant.

Figs. 5A-5E illustrate a range of activity that is possible in terms of device manipulation before such release. In succession, these views show progressive stent frame deployment and steps toward complete recapture. Fig. 5A pictures (literally, given that the figures are based on photographs) the beginning of stent frame deployment as sheath 210 is withdrawn and a distal end 30 of the stent self-expands. Fig. 5B shows the sheath fully withdrawn and full tension on the draw lines/filaments, maintaining a proximal side 32 of the stent 20 in a compresses state. As in Fig. 5D illustrating the same (but in the case of Fig. 5D re-compression after the relaxation of draw lines to allow expansion as in Fig. 5C), the sheath can be advanced to fully recapture the stent frame. With the beginning of such action shown in Fig. 5E, the stent frame can be fully recovered within sheath 210 -- whether for the purpose of repositioning or bulk retrieval of the device.

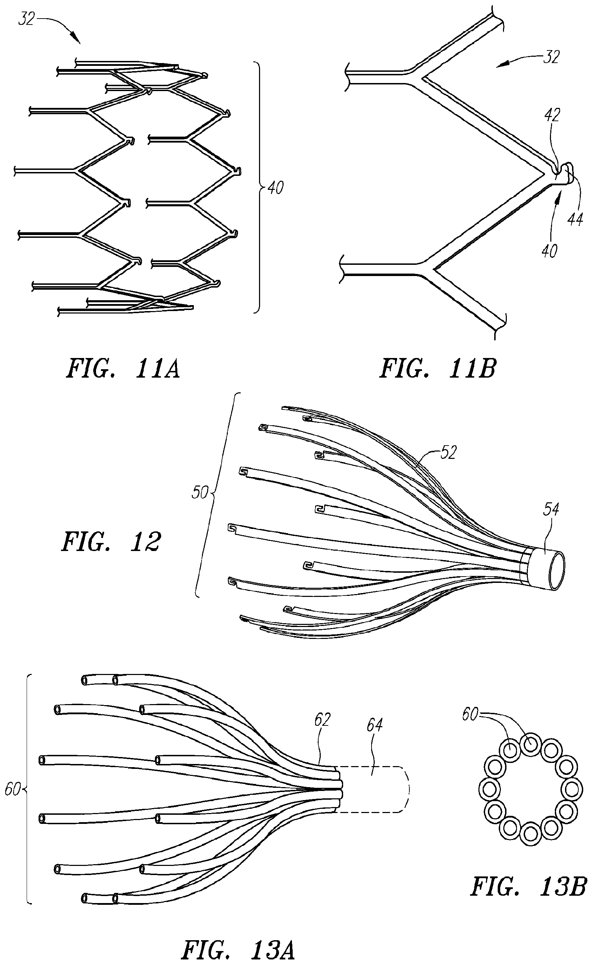

Another delivery device example is able to offer similar advantages in terms of delivery, repositioning and/or percutaneous retrieval. Stent frame components of such a system are shown in Figs. 11A and 11B. In each view, a proximal end 32 of a stent frame 20 includes clasp features 40. Each clasp may comprise a bridge section 42 and an overhang section 44. Complementary clasp features 50 are provided at the end of resilient retainer "arms" or "fingers" 52 associated with a delivery system pusher. These arms may comprise Nitinol or another elastic/superelastic material. They are biased outward such that they spring out to a position as shown in Fig. 12 when released from restraint (e.g., upon exiting a delivery system sheath element or delivery/guide catheter body). Arms 52 are joined or meet at a hub 54. These components may be cut from a single hypotube or polymer sleeve that extends to the proximal end of the delivery system (not shown) as one piece or be assembled using conventional techniques such as laser welding, etc. In any case, pairs of complementary clasp elements 40/50 are releasably engaged in sheaths 60.

Figs. 13A and 13B illustrate a construct in which multiple sheaths 60 extend to and join at a hub 62 optionally extending proximally as a single sleeve 64. Such a structure can be produced by bundling and reconfiguring/fusing a plurality of thermoplastic sheaths, bundling and bonding a plurality of sheaths, and splitting a end of a multi-lumen extrusion into a plurality of separate sheaths. Other means of construction will be appreciated by those with skill in the art as well.

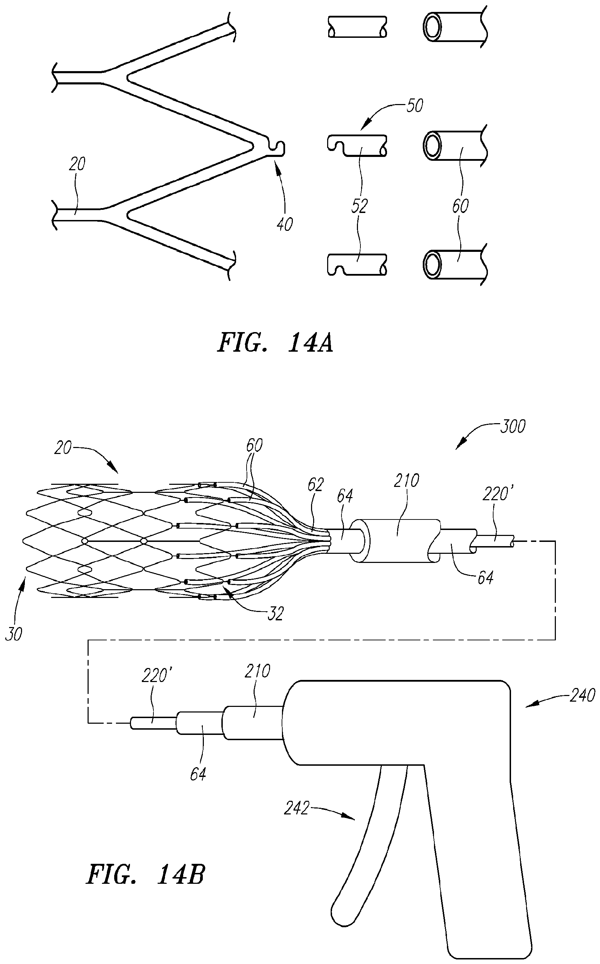

Regardless, Fig. 14A provides a partial assembly drawing illustrating the axial alignment for a plurality of interfacing members. Fig. 14B shows the same components of the device shown in Figs. 11A and 11B brought together in a completed apparatus assembly 300. As in the embodiments above, such a system may optionally include a cover sheath 210 and a handle 240. In addition, system 300 may include an innermost elongate sleeve 220' optionally providing a lubricious PTFE liner for a guidewire lumen and/or column or "push" strength to the system.

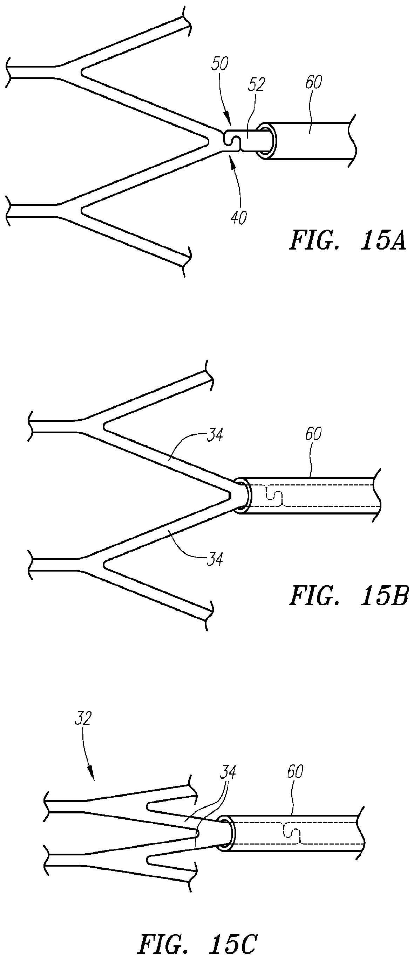

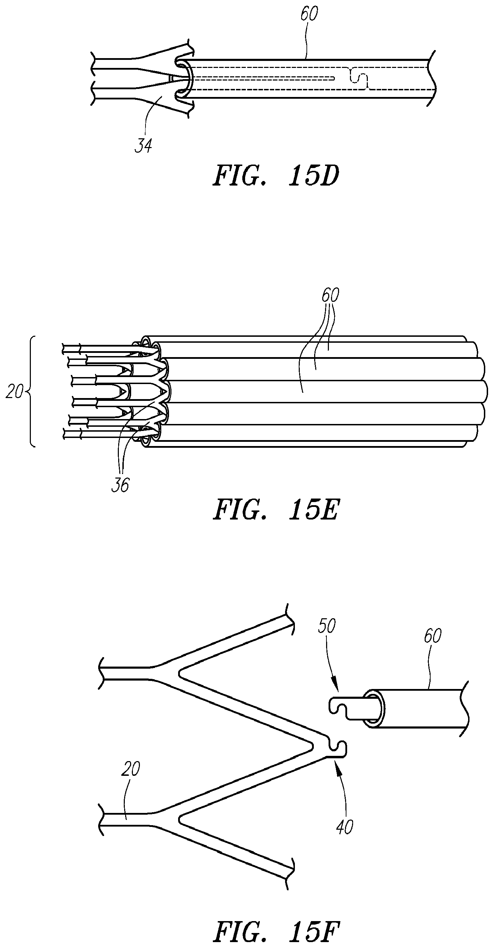

Figs. 15A-15F illustrate the operation of an intended interaction of the subcomponents of system 300. In Fig. 15A, the heart valve frame clasp/link 40 is interfaced with clasp/line 50. In Fig. 15B, clasps features 40/50 are trapped within sheath 60. At this point, further withdrawal of stent frame 20 into sheath element 60 or (stated otherwise) advancement of sheath 60 over adjacent proximal stent struts 34 results in a condition as shown in Fig. 15C. Here, struts 34 are brought together collapsing the entirety of the proximal end 32 of stent frame 20 (given that the same condition is achieve around the entire periphery of the stent by paired device features). As shown in Fig. 15D, sheath 60 can cover the entirety of struts 34 up to their junctions 36 with adjacent struts. The net effect is shown in Fig. 15E where the entire proximal side of the stent frame 20 is compressed efficiently by the multiple sheath elements shown.

As summarized above, the zip tub part assembly (sheaths 60 and associated components) may be variably retracted to allow the proximal end 32 of the stent frame to partially expand or retracted sufficiently to allow the stent frame to fully expand. Alternatively, the zip part/assembly may be secured in position and the arm retainer 54 retracted to variably collapse the proximal end of the heart valve device (up to fully collapsed) or variably advanced to allow the self-expanding heart valve device to variably expand (up to fully expanded). Further action associated with collapse/compression and expansion of the stent frame is achieved by covering and uncovering the stent frame with optional sheath 210 or by a guide catheter.

In any case, upon achieving desired implant placement, clasp elements 40/50 can be freed from confinement within the sheath member(s) 60 thereby unlinking the elements allowing stent frame 20 release as shown in Fig. 15F and allowing delivery system withdrawal from a patient in a successful percutaneously heart valve implantation procedure.

In the various delivery system architectures, the catheter/pusher shaft or sleeve may comprise a simple extrusion (e.g., PTFE, FEP, PEEK, PI etc.) or may be constructed using conventional catheter construction techniques and include a liner, braid support and outer jacket (not shown). Likewise, the various tubular members may comprise extrusion (per above), metal hypotube, etc. Further, the stent frame may be constructed using conventional laser cutting and electropolishing techniques and/or be otherwise constructed. In embodiments intended for tracking through a guide/delivery catheter without an incorporated sheath, a loading sheath may be provided over the implant. Advantageously, any such loading sheath is splittable. Other typical percutaneous access instruments (such as wires, etc.), valves, and other hardware may also be employed in connection with the invention embodiments.

The related methods may include each of the physician activities associated with implant positioning, re-positioning, retrieval and/or release. Regarding these methods, including methods of manufacture and use, these may be carried out in any order of the events which is logically possible, as well as any recited order of events.

Though the invention has been described in reference to several examples, optionally incorporating various features, the invention is not to be limited to that which is described or indicated as contemplated with respect to each variation of the invention. Various changes may be made to the invention described without departing from the scope of the invention as defined by the apended claims.

The breadth of the different inventive embodiments or aspects described herein is not to be limited to the examples provided and/or the subject specification, but rather only by the scope of the issued claim language.

## Claims

1. A medical device (200) for implant delivery, comprising:
an elongate sleeve (220), a plurality of apertures (222) formed in the sleeve (220) adjacent a distal end (224);
a plurality of filaments (230), each filament received within the sleeve (220) from a proximal end, passing out of the distal end (224), looping over said distal end (224) of the sleeve (220), passing through one of the apertures (222) and through the sleeve (220) to a proximal end; and
an elastic self expanding stent frame (20) comprising a plurality of struts (28) each strut (28) including a proximal aperture (22);
wherein each filament (230) passes through one of said proximal apertures (22) and runs along a length of the strut (28),
wherein the medical device (200) is adapted for controlling expansion and contraction of the stent frame (20) by varying the position and/or tension applied to the the filaments (230).

2. The device of claim 1, wherein the stent frame (20) comprises distal apertures (24) paired with proximal apertures (22), wherein each of filaments (230) pass through one of said distal apertures (24).

3. The device of claim 1 or claim 2, further comprising a valve (10) connected to the stent frame (20).

4. The device of any preceding claim, wherein the sleeve (220) comprises a distal shoulder (226) for abutting the stent frame (20) during advancement.

5. The device of any preceding claim, further comprising a sheath (210) to cover the stent frame (20).

6. The device of claim 5, where the sheath (210) is connected to a handle (240) adapted to advance the sheath (210) to cover the stent frame (20) or retract the sheath (210) to uncover the stent frame (20).

7. The device of claim 6, wherein the handle (240) is adapted to select and actuate the sheath (210) to intermediate states between full advancement and full retraction.

8. The device of claim 7, wherein retraction of the sheath (210) allows a distal end (30) of the stent frame (20) to expand, and advancement of the sheath (210) compress a distal portion of the stent frame (20).

9. The device of any preceding claim, wherein two ends of each filament (230) pass through the proximal end of the sleeve (220).

10. The device of claim 9, wherein the two ends are connected to a handle adapted to actuate the filaments (230).

11. The device of claim 10, wherein the handle is adapted to select and actuate a proximal portion of the stent frame (20) between states of full expansion for deployment and full compression for retrieval.

12. The device of claim 11, wherein the handle is adapted to select and actuate the proximal end of the stent frame (20) to intermediate states between full compression and full expansion.

## Patentansprüche

1. Eine medizinische Vorrichtung (200) zur Implantatfreilegung, die Folgendes beinhaltet:
eine längliche Hülse (220), eine Vielzahl von Öffnungen (222), gebildet in der Hülse (220) neben einem distalen Ende (224);
eine Vielzahl von Filamenten (230), wobei jedes Filament innerhalb der Hülse (220) von einem proximalen Ende aufgenommen wird, aus dem distalen Ende (224) herausläuft,
sich über das distale Ende (224) der Hülse (220) schlingt, durch eine der Öffnungen (222) und durch die Hülse (220) zu einem proximalen Ende verläuft; und
einen elastischen, selbst expandierenden Stentrahmen (20), der eine Vielzahl von Streben (28) beinhaltet, wobei jede Strebe (28) eine proximale Öffnung (22) umfasst;
wobei jedes Filament (230) durch eine der proximalen Öffnungen (22) verläuft und entlang einer Länge der Strebe (28) verläuft,
wobei die medizinische Vorrichtung (200) durch das Variieren der Position und/oder Spannung, die auf die Filamente (230) angewendet wird, zum Steuern von Expansion und Kontraktion des Stentrahmens (20) angepasst ist.

2. Vorrichtung gemäß Anspruch 1, wobei der Stentrahmen (20) distale Öffnungen (24) beinhaltet, die mit proximalen Öffnungen (22) gepaart sind, wobei jedes der Filamente (230) durch eine der distalen Öffnungen (24) verläuft.

3. Vorrichtung gemäß Anspruch 1 oder Anspruch 2, die ferner ein Ventil (10) beinhaltet, das mit dem Stentrahmen (20) verbunden ist.

4. Vorrichtung gemäß einem der vorhergehenden Ansprüche, wobei die Hülse (220) eine distale Schulter (226) zum Anstoßen an den Stentrahmen (20) während des Vorrückens beinhaltet.

5. Vorrichtung gemäß einem der vorhergehenden Ansprüche, die ferner eine Hülle (210) beinhaltet, um den Stentrahmen (20) zu bedecken.

6. Vorrichtung gemäß Anspruch 5, wobei die Hülle (210) mit einem Griff (240) verbunden ist, der angepasst ist, um die Hülle (210) vorzurücken, um den Stentrahmen (20) abzudecken oder die Hülle (210) zurückzuziehen, um den Stentrahmen (20) freizulegen.

7. Vorrichtung gemäß Anspruch 6, wobei der Griff (240) angepasst ist, um die Hülle (210) in Zwischenzuständen und zwischen vollständigem Vorrücken und vollständigem Zurückziehen auszuwählen und zu betätigen.

8. Vorrichtung gemäß Anspruch 7, wobei das Zurückziehen der Hülle (210) einem distalen Ende (30) des Stentrahmens (20) das Expandieren und das Vorrücken der Hülle (210) das Komprimieren eines distalen Abschnitts des Stentrahmens (20) ermöglicht.

9. Vorrichtung gemäß einem der vorhergehenden Ansprüche, wobei zwei Enden jedes Filaments (230) durch das proximale Ende der Hülse (220) verlaufen.

10. Vorrichtung gemäß Anspruch 9, wobei die zwei Enden zu einem Griff verbunden sind, der angepasst ist, um die Filamente (230) zu betätigen.

11. Vorrichtung gemäß Anspruch 10, wobei der Griff angepasst ist, um einen proximalen Abschnitt des Stentrahmens (20) zwischen Zuständen vollständiger Expansion zum Entfalten und vollständiger Komprimierung zur Entnahme auszuwählen und zu betätigen.

12. Vorrichtung gemäß Anspruch 11, wobei der Griff angepasst ist, um das proximale Ende des Stentrahmens (20) in Zwischenzuständen zwischen vollständiger Komprimierung und vollständiger Expansion auszuwählen und zu betätigen.

## Revendications

1. Un dispositif médical (200) destiné à la mise en place d'implant, comprenant :
un manchon allongé (220), une pluralité d'ouvertures (222) étant pratiquées dans le manchon (220) adjacentes à une extrémité distale (224) ;
une pluralité de filaments (230), chaque filament étant reçu à l'intérieur du manchon (220) par une extrémité proximale, ressortant en passant par l'extrémité distale (224), faisant une boucle par-dessus ladite extrémité distale (224) du manchon (220), passant dans l'une des ouvertures (222) et dans le manchon (220) jusqu'à une extrémité proximale ; et
une armature formant stent (20) à auto-expansion élastique comprenant une pluralité d'entretoises (28), chaque entretoise (28) incluant une ouverture proximale (22) ;
chaque filament (230) passant dans l'une desdites ouvertures proximales (22) et s'étendant sur une longueur de l'entretoise (28),
le dispositif médical (200) étant conçu pour contrôler l'expansion et la contraction de l'armature formant stent (20) en faisant varier la position et/ou la tension appliquée sur les filaments (230).

2. Le dispositif de la revendication 1, dans lequel l'armature formant stent (20) comprend des ouvertures distales (24) appariées avec des ouvertures proximales (22), chacun des filaments (230) passant dans l'une desdites ouvertures distales (24).

3. Le dispositif de la revendication 1 ou de la revendication 2, comprenant en sus une valvule (10) raccordée à l'armature formant stent (20).

4. Le dispositif de n'importe quelle revendication précédente, dans lequel le manchon (220) comprend un épaulement distal (226) destiné à s'abouter à l'armature formant stent (20) au cours de l'avancement.

5. Le dispositif de n'importe quelle revendication précédente, comprenant en sus une gaine (210) pour couvrir l'armature formant stent (20).

6. Le dispositif de la revendication 5, où la gaine (210) est raccordée à une poignée (240) conçue pour faire avancer la gaine (210) afin de couvrir l'armature formant stent (20) ou pour faire se rétracter la gaine (210) afin de découvrir l'armature formant stent (20).

7. Le dispositif de la revendication 6, dans lequel la poignée (240) est conçue pour sélectionner et actionner la gaine (210) sur des états intermédiaires entre plein avancement et pleine rétraction.

8. Le dispositif de la revendication 7, dans lequel la rétraction de la gaine (210) permet l'expansion d'une extrémité distale (30) de l'armature formant stent (20), et l'avancement de la gaine (210) comprime une portion distale de l'armature formant stent (20).

9. Le dispositif de n'importe quelle revendication précédente, dans lequel deux extrémités de chaque filament (230) passent dans l'extrémité proximale du manchon (220).

10. Le dispositif de la revendication 9, dans lequel les deux extrémités sont raccordées à une poignée conçue pour actionner les filaments (230).

11. Le dispositif de la revendication 10, dans lequel la poignée est conçue pour sélectionner et actionner une portion proximale de l'armature formant stent (20) entre des états de pleine expansion pour le déploiement et de pleine compression pour la récupération.

12. Le dispositif de la revendication 11, dans lequel la poignée est conçue pour sélectionner et actionner l'extrémité proximale de l'armature formant stent (20) sur des états intermédiaires entre pleine compression et pleine expansion.
